# EUROPEAN PATENT SPECIFICATION

(11) **EP 2 382 946 B1**
(45) Date of publication and mention of the grant of the patent: **27.07.2016**
(21) Application number: 09836061.3
(22) Date of filing: 29.12.2009
(51) Int. Cl.: A61F 2/36, A61B 17/32

(54) **REPLACING MODULE FOR FEMORAL HEAD**
ERSATZMODUL FÜR DEN OBERSCHENKELKOPF
MODULE DE REMPLACEMENT DE LA TÊTE FÉMORALE

(30) Priority: 30.12.2008 CN 200810208249
(43) Date of publication of application: 02.11.2011
(73) Proprietor: Shih, Lu-Sun, Taiwan (CN); Yeh, Ching-tao, Taiwan (CN)
(72) Inventor: Shih, Lu-Sun, Taiwan (CN); Yeh, Ching-tao, Taiwan (CN)
(74) Representative: Zeitler Volpert Kandlbinder Patent- und Rechtsanwälte Partnerschaft mbB
(86) International application number: PCT/CN2009/076198
(87) International publication number: WO 2010/075774

(56) References cited:
- EP-A1- 1 437 099
- CN-Y- 2 318 995
- CN-Y- 2 430 959
- CN-Y- 2 534 997
- CN-Y- 2 616 187
- CN-Y- 2 936 174
- CN-Y- 201 399 013
- TW-A- 201 023 816
- US-A- 2 668 531
- US-A- 5 376 126
- US-A1- 2005 277 943
- US-A1- 2008 004 711
- US-A1- 2008 004 711
- US-B1- 6 488 716

## Description

### BACKGROUND OF THE INVENTION

The present invention relates to a femoral head prosthesis assembly With operation instruments, an operator can more precisely perform femoral head replacement and more securely bond the artificial femoral head with the natural femoral neck to promote operation quality.

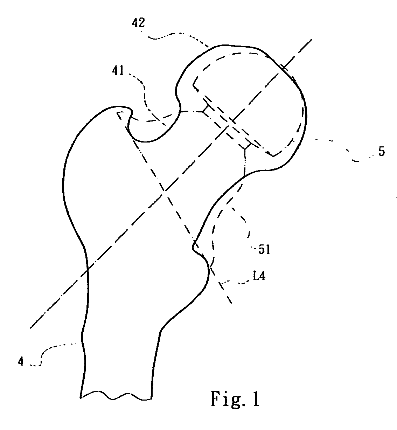

It is known that a stem-type femoral prosthesis or a stemless femoral prosthesis is used in a traditional femoral head replacement. As shown in Fig. 1, the necrotic or worn femoral head 42 along with the femoral neck 41 is generally directly cut off from the femur 4 along an oblique cut t ing line L4 marked on the root of the femoral neck 41. Then the femoral medulla is extracted to ream the femoral medullary cavity. Thereafter, an artificial femoral stem is implanted in the femoral medullary cavity and an artificial femoral head 5 is connected to the stem. Such operation is disclosed in U.S. Patent No. 5163961, entitled COMPRESSION-FIT HIP PROSTHESIS AND PROCEDURE FOR IMPLANTATION THEREOF. Also, Chinese Utility Model Patent Publication No. CN2430959Y discloses a self-locked stemless hip prosthesis. According to this patent, the necrotic or worn femoral head 42 is cut off from the femoral neck and a cap body 51 is fixed on the remaining femoral neck with bolts. In practice, the above operations have some defects as follows:
1. With respect to traditional hemiarthroplasty in which a stem-type femoral prosthesis is used, it is necessary to cut off an extensive part of the femoral head and extract a large amount of femoral medulla for hemiarthroplasty. Therefore, the extent of trauma, the operation time and the blood loss are all greatly increased. This significantly increases operation risk as a whole.
2. It is hard to precisely control the dimension of the part of the femur, (such as the femoral neck), that needs to be cut off or extracted. Therefore, it is difficult to precisely match the surface of the stem-type femoral prosthesis or the inner surface of the cap body of the stemless femoral prosthesis with the surface of the remaining part of the femur. As a result, the femoral prosthesis can hardly securely bond with the rigid remaining part or the femoral neck of the femur. Moreover, stress concentration is likely to take place to directly and seriously affect operation result and recovery quality.
3. Every femoral prosthesis has a lifetime. Therefore, after a long period of use, the femoral prosthesis tends to wear, loosen or sink and may need revision. With respect to traditional hemiarthroplasty in which a stem-type femoral prosthesis is used, an extensive part of the femoral head is cut off in the primary operation so that the extent of trauma is pretty great. Consequently, in the revision, it is generally necessary to re-ream the femoral medullary cavity. Such operation inevitably increases the extent of trauma and is more difficult to perform.
4. With respect to traditional hemiarthroplasty in which a stemless femoral prosthesis is used, there is no scientific and precise measuring method or program for the part to be cut off. Therefore, the cutting position of the femoral neck is often decided simply by experience. Accordingly, the operation result apparently varies with the operators and it is hard to ensure good operation quality. Moreover, it is impossible to perform pre-operational evolution to simulate and evaluate the operation. Therefore, it is hard to give concrete pre-operation metal construction to a patient.
5. As shown in Fig. 1, the cap body 51 of the stemless femoral prosthesis is obliquely positioned on the cutting plane taken along the cutting line L4. In this case, it cannot be sure that the surface of the femoral neck 41 continuously tightly fits with the inner surface of the cap body 51. Consequently, the stress applied to the hip joint can be hardly uniformly distributed and transmitted and stress concentration is likely to take place in practical use. This affects durability of the stemless femoral prosthesis and needs improvement.

From EP 1 437 099 A1 a device for protecting femoral neck is known which comprises a main body having a hollow sleeve corresponsive to a trimmed femoral neck wherein said sleeve on the rim extends a flange that connects a femur and a femoral neck, at the other end of the rim a neck extends, and on the top of said neck a bolt is fixed to penetrate the femur neck in the sleeve.

### SUMMARY OF THE INVENTION

It is therefore a primary object of the present invention to provide a femoral head prosthesis assembly, which can be more precisely assembled and implanted to improve revision effect for hip joint and enhance operation quality.

It is a further object of the present invention to provide the above femoral head prosthesis assembly, which is directly fixedly assembled with the femoral neck by a maximum area. A major part of the femoral neck is reserved without reaming the femoral medullary cavity. Accordingly, in case a revision is needed, a traditional hemiarthroplasty can be further performed to cut off the femoral neck and implant a stem-type femoral prosthesis in the femur. Therefore, the lifetime of the femur can be prolonged.

It is still a further object of the present invention to provide the above femoral head prosthesis assembly, which can be tightly fitted on the femoral neck and fully attached to the surface of the proximal end thereof. Moreover, the femoral head prosthesis assembly extends to the distal end, that is, the root portion of the femoral neck to replace the removed proximal end thereof. The femoral head prosthesis assembly contacts the surface of the remaining rigid root portion of the femoral neck by a maximum area so that the femoral head prosthesis assembly can bond with the remaining part of the femoral neck by enhanced strength. Accordingly, the femoral head prosthesis assembly can be securely bonded with the natural femoral neck with longer lifetime.

Further disclosed is an operation instrument for femoral head replacement. The operation instrument is precisely made on the basis of data achieved by means of scanning the surface of the femoral neck of a patient with a medical volume scanning equipment. The operation instrument is manufactured in accordance with biological dynamics to reduce cutting area of the femur and thus lower trauma caused by the operation. Moreover, with cooperative simulation software, the data can be input to a computer for an operator to perform a pre-operational evolution to simulate the operation and get experienced with various situations in the replacement procedure. Accordingly, the success possibility of the operation can be enhanced to have a patient have concrete confidence before the operation and thus lower operation risk.

According to the above objects, the femoral head prosthesis assembly of the present invention includes a cap body and an artificial femoral head. The cap body has a caved portion and a connection portion, which extend in opposite directions. The caved portion has a root section and a top section. The root section has an inner surface with a configuration and a dimension achieved by means of scanning the surface of the femoral neck of a patient with a medical volume scanning equipment. Those points of the periphery of the femoral neck that are spaced from a central line of the femoral neck by shortest distances are taken to compose a cutting loop defining a narrowest cross section. The top sect ion has an inner surface with a configuration and a dimension adapted to those of the narrowest cross section. Accordingly, the top section can be tightly fitted on a protrusion portion formed on the femoral neck by cutting the femoral neck and the inner surface of the cap body can be tightly bonded with and located on the surface of the femoral neck. The artificial femoral head has a socket for fixedly fitting on the connection portion of the cap body.

The operation instruments for femoral head replacement of the present invention include a femoral neck holder and a shaper blade. The femoral neck holder is a hollow casing with a configuration adapted to the surface configuration of the femoral neck. The femoral neck holder is sectioned at a middle port ion thereof to form a side opening. A guide tube is disposed on a top face of the femoral neck holder. The guide tube extends along the central line of the femoral neck for forming a central hole on the femoral neck. A transverse slot is formed on the middle portion of the femoral neck holder for indicating a cutting position. The shaper blade has a sleeve portion and a cutting blade portion. The cutting blade portion has a lower opening. A periphery of the opening is formed with a sharp blade section. The blade section has a dimension and a sectional configuration in conformity to those of a cutting loop composed of points of the periphery of the femoral neck, which points are spaced from a central line of the femoral neck by shortest distances. A guide member is disposed in a central hole of the femoral neck. The sleeve portion is fitted around the guide member to reciprocally move along the guide member for cutting the femoral neck into a predetermined configuration.

With the femoral head prosthesis assembly of the present invention, the operation quality is promoted, the lifetime of the artificial femoral head is prolonged and the operation risk is reduced.

The present invention can be best understood through the following description and accompanying drawings wherein:

### BRIEF DESCRIPTION OF THE DRAWINGS

Fig. 1 is a view showing that a conventional artificial femoral head is implanted in a femur, also showing the most often seen femoral head cutting line in the traditional hip arthroplasty;
Fig. 2 is a view showing the primary cutting line of the femoral head and other relevant locating lines according to the present invention;
Fig. 3 is a view showing the cutting loop and replacement loop of the femoral neck according to the present invention;
Fig. 4 is a perspective view of a femoral neck holder;
Fig. 5 is a top view of the femur after the femoral head is cut off along the primary cutting line;
Fig. 6 is a perspective view of the femur after the femoral head is cut off along the primary cutting line;
Fig. 7 is a view showing that the femoral neck holder is capped on the end section of the femur after primary cutting;
Fig. 8 is a perspective view of the shaper blade;
Fig. 9 is a sectional view of the shaper blade;
Fig. 10 shows the operation of the shaper blade;
Fig. 11 is a view showing a conic protrusion portion is formed on the femoral neck after cut with the shaper blade;
Fig. 12 is a perspective exploded view of the artificial femoral head assembly of the present invention;
Fig. 13 is a perspective assembled view of the artificial femoral head assembly of the present invention;
Fig. 14 is a side sectional assembled view of the artificial femoral head assembly of the present invention;
Fig. 15 is a perspective assembled view of the artificial femoral head assembly of the present invention as seen from another side;
Fig. 16 shows that the artificial femoral head of the present invention is housed in a cup implanted in an acetabulum; and
Fig. 17 is a perspective view of another embodiment of the artificial femoral head assembly of the present invention.

- 1: femoral neck holder
- 10: bottom portion
- 11: side opening
- 12: plane face
- 13: guide tube
- 131, 211: guide hole 131
- 132: drilling instrument
- 14, 132: lower periphery
- 15: locating hole
- 151: temporary retainer member
- 16: transverse slot
- 161: longitudinal slot
- 2: shaper blade
- 21: sleeve portion
- 22: cutting blade section
- 221: blade section
- 23: guide member
- 3: artificial femoral head assembly
- 31, 34: cap body
- 311: caved portion
- 3111: root section
- 3112: top portion
- 313: connection portion
- 314: through hole
- 315: locating hole
- 316: auxiliary locating member
- 32: locating member
- 33, 5: artificial femoral head
- 331: socket
- 341: fixing extension portion
- 342: locating members(bolts)
- 35: cup
- 4, 40, 400: femur
- 41: femoral neck
- 42: femoral head
- 43: central hole
- 44: protrusion portion
- 51: cap body
- C1: cutting loop
- C2: replacement loop
- CL: common central line
- L2: femoral head-neck interface line
- L3: primary cutting line
- L4: cutting line
- P2: cutting face
- P3: primary cutting face

### DETAILED DESCRIPTION OF THE PREFERRED EMBODIMENTS

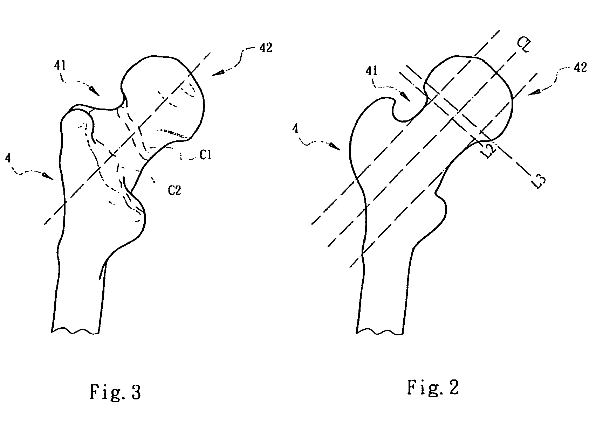

Please refer to Figs. 2 and 3. Before the artificial femoral head replacement, as seen from one side, a femoral head-neck interface line L2 is properly taken at the junction between the femoral head 42 and femoral neck 41 of the femur 4. In addition, a primary cutting line L3 is taken in parallel to the femoral head-neck interface line L2. The primary cutting line L3 is spaced from the femoral head-neck interface line L2 in a direction to the femoral head 42 by a certain distance. A common central line CL of the femoral head 42 and femoral neck 41 is determined on the basis of the femoral head-neck interface line L2 or the primary cutting line L3. Also, the data of surface configuration of the femoral neck 41 are measured by means of a medical volume (three-dimensional) scanning equipment such as a computer tomography (CT) or a magnetic resonance imaging (MRI) equipment. Then, the shortest distances from the central line CL to the periphery of the femoral neck 41 are taken to obtain a cutting loop C1 defining a narrowest cross section. In addition, a maximum junction cross section is taken below the cutting loop C1 between the root of the femoral neck 41 and the femur 4. The periphery of the cross section forms a replacement loop C2.

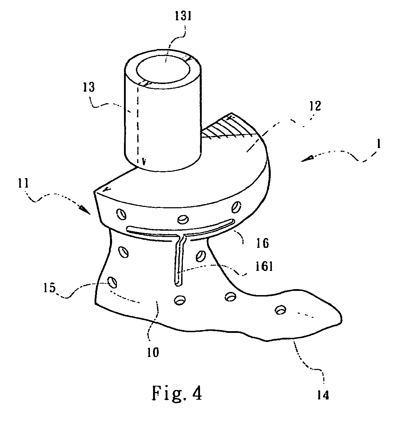

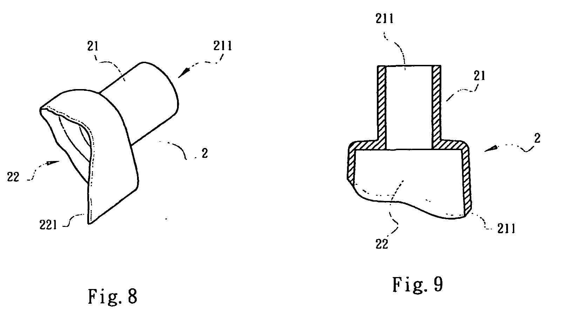

Referring to Figs. 4 to 11, the operation instruments mainly include a femoral neck holder 1 and a shaper blade 2. The femoral neck holder 1 essentially has a configuration, which is adapted to the position of the primary cutting line L3 in conformity to the surface configuration of the periphery of the femoral neck 41. The femoral neck holder 1 has a bottom portion 10 positioned under the cutting loop C1. The bottom portion 10 has an inner surface with a configuration and a dimension in conformity to or approximately in conformity to those of the outer surface of the femoral neck 41. In addition, the femoral neck holder 1 has a top plane face 12 corresponding to the primary cutting line L3. The top plane face 12 has a dimension not less than that of a cross section taken along the primary cutting line L3. The femoral neck holder 1 is longitudinally sectioned at a middle portion thereof to form a side opening 11. A guide tube 13 is disposed on the top plane face 12. The guide tube 13 has a guide hole 131 extending along the central line CL. A transverse slot 16 is formed on the middle portion of the femoral neck holder 1 beside the top plane face 12 in alignment with the femoral head-neck interface line L2. In addition, a longitudinal slot 161 is formed on the femoral neck holder 1 and extends from a middle point of the transverse slot 16 in parallel to the central line CL. Multiple locating holes 15 are further distributed over the femoral neck holder 1. A lower periphery 14 of the bottom portion 10 of the femoral neck holder 1 has a configuration adapted to that of the replacement loop C2 (as shown in Fig. 4). The shaper blade 2 is composed of a sleeve portion 21 and a cutting blade portion 22 connected with the sleeve portion 21. The cut t ing blade portion 22 is a hollow portion having a lower opening and an inner diameter tapered from the opening. Accordingly, the cutting blade portion 22 has a substantially conic configuration. The cutting blade portion 22 has a periphery formed with a sharp blade section 221. The blade section 221 has a configuration and a dimension in conformity to those of the cutting loop C1 (as shown in Figs. 8 and 9).

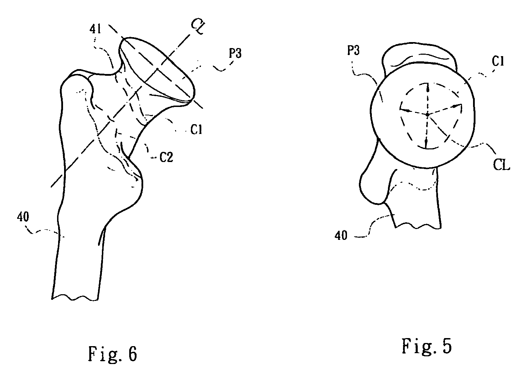

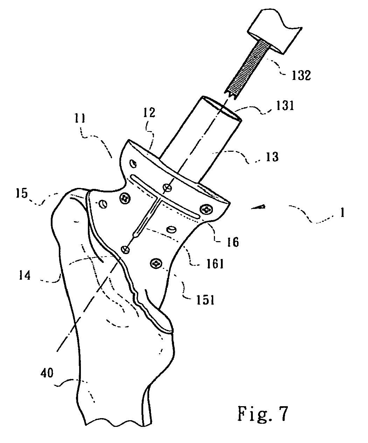

In operation, the femoral head 42 is first cut along the primary cutting line L3 to form a primary cutting face P3 on the femur 40 (as shown in Figs. 5 and 6). The femoral neck holder 1 is capped on the femoral neck 41 through the side opening 11. Multiple temporary retainer members 151 are passed through the locating holes 15 to penetrate into the femur 40 so as to firmly fix the femoral neck holder 1 on the peripheral portion of the femoral neck 41 (as shown in Fig. 7). Thereafter, wi th the indicat ion of the transverse slot 16, an operator can be sure of the t rue position of the femoral head-neck interface line L2. At the same time, the position of the central line L2 can be marked by means of the longitudinal slot 161. A drilling instrument 132 is extended into the guide hole 131 of the guide tube 13 to drill a central hole 43 on the primary cutting face P3 along the central line CL. Then, through the transverse slot 16, the femoral head is cut off along the femoral head-neck interface line L2.

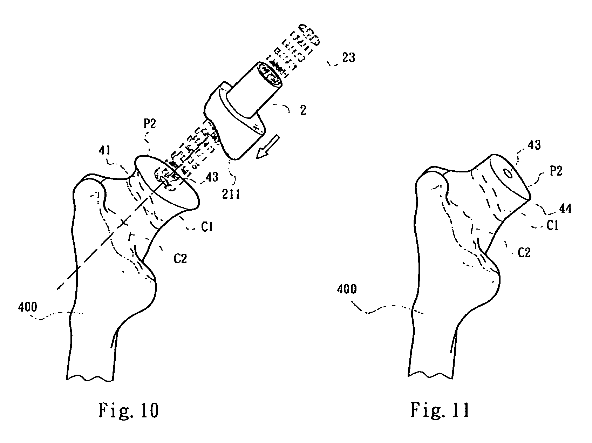

Thereafter, a guide member 23, (which can be a bar body), is inserted and locked in the central hole 43. The sleeve port ion 21 of the shaper blade 2 is fitted around an outward extending portion of the guide member 23. The shaper blade 2 can be reciprocally moved along the guide member 23 (as shown in Fig. 10), whereby the blade section 221 of the cut t ing blade port ion 22 can cut and mill off the periphery of the femoral neck 41 above the cutting loop C1 to form a substantially conic protrusion portion 44 with a central hole 43. The protrusion portion 44 is tapered from lower side to upper side and has a cross section approximately identical to the narrowest cross section formed along the cutting loop C1 on the femoral neck(as shown in Fig. 11). When necessary, the protrusion portion 44 can be modified as tapered shape from lower side to upper side has a substantially conic configuration.

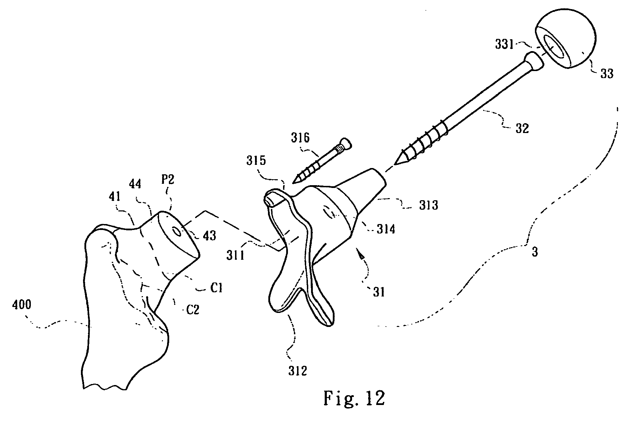

Referring to Figs. 12 to 15, the femoral head prosthesis assembly, (that is, the artificial femoral head assembly 3), of the present invention includes a cap body 31 and an artificial femoral head 33. The cap body 31 has a caved portion 311 and a connection portion 313, which extend in opposite directions and communicate with each other through a through hole 314. The surface configuration data between the cutting loop C1 and the replacement loop C2 are taken from the surface configuration data of the femoral neck 41 as the data of the inner surface configuration of a root section 3111 of the caved portion 311. The caved portion 311 further has a top section 3112 above the root portion 3111. The configuration and size of the surface of the top portion 3112 are adapted to those of the protrusion portion 44, which is tapered from the cutting loop C1 to the femoral head-neck interface line L2 and has a profile approximately identical to that of the narrowest cross section of the femoral neck. A lower periphery 312 of the cap body 31 has a configuration in conformity to that of the replacement loop C2. In addition, at least one locating hole 315 is formed on one side of the caved portion 311 as necessary (as shown in Fig. 12).

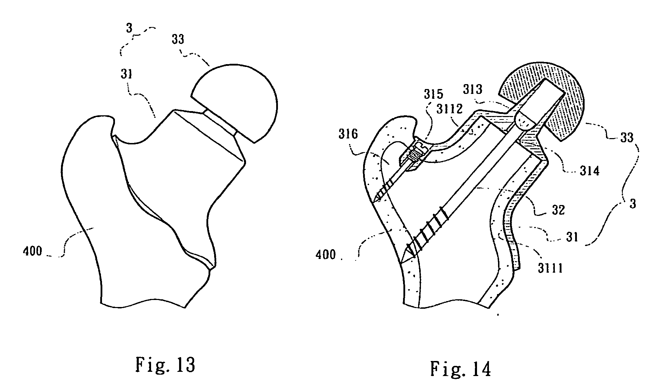

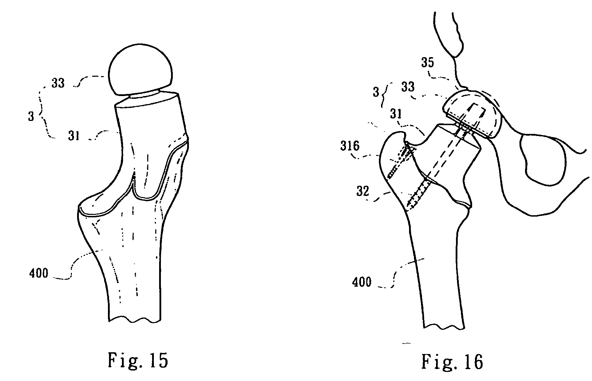

When assembled, the cap body 31 is capped onto a replacement end of the femur 400 with the protrusion portion 44. At this time, the protrusion portion 44 is snugly fitted into the top section 3112 of the cap body 31 with the root section 3111 snugly attaching to the surface of the femoral neck between the cutting loop C1 and the replacement loop C2 (as shown in Figs. 13 and 14). Under such circumstance, the cap body 31 can be highly snugly attached to and securely fitted on the femoral neck. Then, a locating member 32 (screw) is passed through the through hole 314 of the cap body 31 and extended (screwed) into the central hole 43 of the protrusion port ion 44. In addition, an auxiliary locating member 316 is passed through the locating hole 315 and extended (screwed) into the femur 400 beside the protrusion portion 44. Accordingly, the cap body 31 is firmly located at the end section of the femur 400. Thereafter, an artificial femoral head 33 with a socket 331 is fitted onto the connection po r t i on 313 of the cap body 31 and fixed thereon (as shown in Fig. 15) to complete the femoral head replacement. In practice, the artificial femoral head 33 can be relatively rotatably housed in a cup 35. In this case, the artificial femoral head assembly 3 and the cup 35 can be respectively implanted in the femur and the acetabulum to replace the necrotic hip joint of a patient.

The femoral neck holder 1 and the shaper blade 2 help an operator to precisely cut, drill and mill the respective parts of the femur so as to enhance assembly precision and improve hip joint revision effect after operation as well as promote operation quality. Moreover, cooperative computer simulation software serves to provide pre-operational evolution for an operator. Accordingly, the operator can previously simulate the operation before it is really performed. In this case, the operator can get experienced with the operation to lower the possibility of error in the operation and ensure success of the operation. Furthermore, the cap body 31 is tightly fitted on the protrusion portion 44 of the top end of the femoral neck 41 and secured with the locating member 32 and auxiliary locating member 316. Therefore, the cap body 31 is more firmly bonded with the femoral neck 41 with better connection strength. The cap body 31 contacts the end section of the femur 400 by maximum effective replacement area. Therefore, the action force transmitted from the artificial femoral head assembly 3 to the femoral neck 41 can be distributed to respective parts of the end section of the femur 400 so as to avoid possible fracture due to stress concentration. Accordingly, the ability of the end section of the femur 400 to bear the action force is greatly enhanced and the lifetime of the entire artificial femoral head assembly 3 can be prolonged. In the traditional operation, the femoral head 42 is directly cut off along the root section of the femoral neck 41. In comparison with the traditional operation, according to the present invention, the femoral head 42 is cut off along the femoral head-neck interface line L2 above the cut t ing loop C1, while the major part of the femoral neck 41 below the cutting loop C1 is reserved. Therefore, in case the cap body 31 or any other component of the artificial femoral head assembly 3 is damaged and a revision is needed, a traditional operation can be further performed (to cut off the femoral neck 41 along the cutting line L4). Accordingly, the lifetime of the femur 400 can be prolonged.

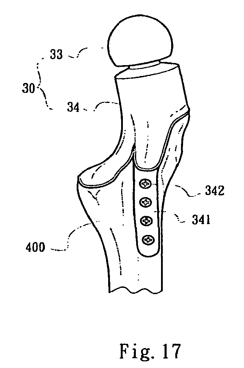

Fig. 17 shows another embodiment of the artificial femoral head assembly 30 of the present invention. In this embodiment, the artificial femoral head assembly 30 includes the same artificial femoral head 33 and another type of cap body 34. The cap body 34 has a structure based on the cap body 31. The cap body 34 is different from the cap body 31 only in that the cap body 34 additionally has a plate-like fixing extension portion 341 extending from the lower periphery of the cap body 34. Multiple locating members 342 (bolts) can be passed through the fixing extension portion 341 to penetrate (screw) into the femur 400. The fixing extension portion 341 serves as a reinforcing structure, which provides enhanced locating effect for the cap body 34, especially for a patient of osteoporosis.

In conclusion, with the femoral head prosthesis assembly of the present invention, the operation quality is promoted, the lifetime of the artificial femoral head is prolonged and the operation risk is reduced. The above embodiments are only used to illustrate the present invention, not intended to limit the scope thereof.

## Claims

1. A femoral head prosthesis assembly comprising:
a cap body (31, 34) having a caved portion (311) and a connection portion (313), which extend in opposite directions, the caved portion (311) having a root section (3111) and a top section (3112), the root section (3111) having an inner surface with a configuration and a dimension achieved by means of scanning the surface of the femoral neck (41) of a patient with a medical volume scanning equipment, points of the periphery of the femoral neck (41), which are spaced from a central line (CL) of the femoral neck (41) by shortest distances being taken to compose a cutting loop (C1) defining a narrowest cross section, the top section (3112) having an inner surface with a configuration and a dimension adapted to those of the narrowest cross section, whereby the top section (3112) can be tightly fitted on a protrusion portion (44) formed on the femoral neck (41) by cutting the femoral neck (41) and the inner surface of the cap body (31,34) can be tightly bonded wi th and located on the surface of the femoral neck (41); and an artificial femoral head (33) fixedly fitted on the connection portion (313) of the cap body (31,34).

2. The femoral head prosthesis assembly as claimed in claim 1, wherein the cap body (31,34) is formed with a central through hole (314), whereby a locating member (32) can be passed through the through hole (314) to extend into a top face of the protrusion portion (44) of the femoral neck (41) for locating the cap body(31,34).

3. The femoral head prosthesis assembly as claimed in claim 1 or 2, wherein the cap body (31,34) is further formed wi th a locating hole (315), whereby an auxiliary locating member (316) can be passed through the locating hole (315) to extend into the femur (4 ,40,400).

4. The femoral head prosthesis assembly as claimed in one of claims 1 to 3, wherein the cap body (31,34) further has at least one plate-like fixing extension portion (341), whereby at least one locating member (342) can be passed through the fixing extension portion (341) to penetrate into the femur (4 ,40,400).

5. The femoral head prosthesis assembly as claimed in one of the foregoing claims, wherein the protrusion portion (44) formed on the femoral neck (41) is tapered from lower side to upper side and has a substantially conic configuration.

6. The femoral head prosthesis assembly as claimed in one of the foregoing claims, wherein a maximum junction cross section is taken between the root of the femoral neck (41) and the femur (4, 40,400), which is defined by a replacement loop (C2), a bottom periphery of the cap body (31, 34) having a configuration in conformity to the replacement loop (C2).

## Patentansprüche

1. Oberschenkelkopf-Prothesenanordnung, umfassend einen Kappenkörper (31, 34) mit einem hohlen Teil (311) und einem Verbindungsteil (313), die sich in entgegengesetzte Richtungen erstrecken, wobei der hohle Teil (311) einen unteren Abschnitt (3111) und einen oberen Abschnitt (3112) aufweist, der untere Abschnitt (3111) mit einer inneren Oberfläche versehen ist, deren Form und Abmessung mit Hilfe einer Einrichtung zum Scannen der Oberfläche des Oberflächenhalses (41) eines Patienten durch eine medizinische Raum-Scan-Ausrüstung ermittelt sind, wobei des weiteren Punkte des Umfangs des Oberschenkelhalses (41), die von einer Mittellinie (CL) des Oberschenkelhalses (41) die kürzesten Abstände haben, beabstandet sind und diese kürzesten Abstände dazu dienen, eine Schnittschleife (C1) zusammenzusetzen, die einen engsten Querschnitt bildet, wobei des weiteren der obere Abschnitt (3112) eine innere Oberfläche mit einer Gestalt und einer Abmessung aufweist, die an diejenigen des engsten Querschnitts angepaßt sind, wodurch der obere Abschnitt (3112) eng auf einen vorstehenden Teil (44) angepaßt werden kann, der auf dem Oberschenkelhals (41) ausgebildet ist, und zwar durch Schneiden des Oberschenkelhalses (41), wodurch die innere Oberfläche des Kappenkörpers (31, 34) dicht mit der Oberfläche des Oberschenkelhalses (41), auf der sie liegt, verbunden werden kann, und wobei schließlich ein künstlicher Oberschenkelkopf (33) auf dem Verbindungsteil (313) des Kappenkörpers (31, 34) fixiert wird.

2. Oberschenkelkopf-Prothesenanordnung nach Anspruch 1, **dadurch gekennzeichnet, daß** der Kappenkörper (31, 34) mit einem zentralen Durchgangsloch (314) versehen ist, und daß ein Verbindungskörper (32) durch das Durchgangsloch (314) gesteckt werden kann, so daß er sich in eine obere Fläche des vorstehenden Teils (44) des Oberschenkelhalses (41) hinein erstreckt, um dadurch den Kappenkörper (31, 34) anzuordnen.

3. Oberschenkelkopf-Prothesenanordnung nach Anspruch 1 oder 2, **dadurch gekennzeichnet, daß** der Kappenkörper (31, 34) des weiteren mit einem Anordnungsloch (315) versehen ist, wodurch ein Hilfsanordnungskörper (316) durch das Anordnungsloch (315) hindurchtreten kann, um sich in das Oberschenkelbein (4, 40, 400) zu erstrecken.

4. Oberschenkelkopf-Prothesenanordnung nach einem der Ansprüche 1 bis 3, **dadurch gekennzeichnet, daß** der Kappenkörper (31, 34) des weiteren wenigstens einen plattenartigen, fixierenden Verlängerungsteil (341) aufweist, wodurch wenigstens ein Anordnungskörper (342) durch den fixierenden Verlängerungsteil (341) gesteckt werden kann, um in das Oberschenkelbein (4, 40, 400) einzutreten.

5. Oberschenkelkopf-Prothesenanordnung nach einem der vorhergehenden Ansprüche, **dadurch gekennzeichnet, daß** der vorspringende Teil (44), der auf dem Oberschenkelhals (41) ausgebildet ist, von der Unterseite zu der Oberseite abgeschrägt ist und eine im wesentlichen konische Gestalt aufweist.

6. Oberschenkelkopf-Prothesenanordnung nach einem der vorhergehenden Ansprüche, **dadurch gekennzeichnet, daß** zwischen dem Boden des Oberschenkelhalses (41) und dem Oberschenkelbein (4, 40, 400) ein maximaler Verbindungsquerschnitt besteht, der durch eine Ergänzungsschleife (C2) und einen unteren Umfang des Kappenkörpers (31, 34) gebildet wird, welcher eine Gestalt aufweist, die zu der Ergänzungsschleife (C2) paßt.

## Revendications

1. Assemblage formant prothèse de tête fémorale comprenant :
un corps de capuchon (31, 34) ayant une portion évidée (311) et une portion de connexion (313), qui s'étendent dans des directions opposées, la portion évidée (311) ayant une section de racine (3111) et une section de sommet (3112), la section de racine (3111) ayant une surface intérieure avec une configuration et une dimension obtenues au moyen d'un scannage de la surface du col fémoral (41) d'un patient avec un équipement de scannage médical volumétrique, des points de la périphérie du col fémoral (41), qui sont espacés d'une ligne centrale (CL) du col fémoral (41) sur les distances les plus courtes, étant pris pour composer une boucle de coupe (C1) définissant une section transversale la plus étroite, la section de sommet (3112) ayant une surface intérieure avec une configuration et une dimension adaptées à celles de la section transversale la plus étroite, grâce à quoi la section de sommet (3112) peut être intimement coiffée sur une portion en projection (44) formée sur le col fémoral (41) en taillant le col fémoral (41), et la surface intérieure du corps de capuchon (31, 34) peut être intimement jointe avec et localisée sur la surface du col fémoral (41), et une tête fémorale artificielle (33), coiffée de manière fixe sur la portion de connexion (313) du corps de capuchon (31, 34).

2. Assemblage formant prothèse de tête fémorale selon la revendication 1, dans lequel le corps de capuchon (31, 34) est formé avec un trou traversant central (314), grâce à quoi un élément de localisation (32) peut être passé à travers le trou traversant (314) pour s'étendre jusque dans une face supérieure de la portion en projection (44) du col fémoral (41) pour localiser le corps de capuchon (31, 34).

3. Assemblage formant prothèse de tête fémorale selon la revendication 1 ou 2, dans lequel le corps de capuchon (31, 34) est en outre formé avec un trou de localisation (315), grâce à quoi un élément de localisation auxiliaire (316) peut être passé à travers le trou de localisation (315) pour s'étendre jusque dans le fémur (4, 40, 400).

4. Assemblage formant prothèse de tête fémorale selon l'une des revendications 1 à 3, dans lequel le corps de capuchon (31, 34) a en outre au moins une portion de fixation en extension (341) similaire à une plaque, grâce à quoi au moins un élément de localisation (342) peut être passé à travers la portion de fixation en extension (341) pour pénétrer jusque dans le fémur (4, 40, 400).

5. Assemblage formant prothèse de tête fémorale selon l'une des revendications précédentes, dans lequel la portion en projection (44) formée sur le col fémoral (41) est effilée depuis le côté inférieur vers le côté supérieur et a une configuration sensiblement conique.

6. Assemblage formant prothèse de tête fémorale selon l'une des revendications précédentes, dans lequel une section transversale de jonction maximum est prise entre la racine du col fémoral (41) et le fémur (4, 40, 400), qui est définie par une boucle de remplacement (C2), une périphérie inférieure du corps de capuchon (31, 34) ayant une configuration en conformité avec la boucle de remplacement (C2).
